# EUROPEAN PATENT SPECIFICATION

(11) **EP 4 129 281 B1**
(45) Date of publication and mention of the grant of the patent: **23.07.2025**
(21) Application number: 21796237.2
(22) Date of filing: 28.04.2021
(51) Int. Cl.: A61K 9/08, A61K 31/05, A61K 31/198, A61K 31/455, A61K 47/02, A61K 47/18, A61K 47/20, A61P 13/12, A61P 43/00

(54) **LIQUID FORMULATION AND PHARMACEUTICAL PRODUCT CONTAINING CILASTATIN**
FLÜSSIGE FORMULIERUNG UND PHARMAZEUTISCHES PRODUKT ENTHALTEND CILASTATIN
FORMULATION LIQUIDE ET PRODUIT PHARMACEUTIQUE CONTENANT DE LA CILASTATINE

(30) Priority: 30.04.2020 JP 2020080017
(43) Date of publication of application: 08.02.2023
(73) Proprietor: Denka Company Limited, Tokyo 103-8338 (JP)
(72) Inventor: HIRAYAMA, Yoshiaki, Tokyo 103-8338 (JP)
(74) Representative: Vossius & Partner Patentanwälte Rechtsanwälte mbB
(86) International application number: PCT/JP2021/016959
(87) International publication number: WO 2021/221101

(56) References cited:
- WO-A1-2010/092446
- WO-A1-2010/092446
- WO-A1-2015/111666
- WO-A1-2018/025248
- WO-A2-2007/124382
- CN-A- 106 176 722
- JP-A- 2009 514 939
- JP-A- 2019 194 173
- JP-A- 2019 507 760
- US-A1- 2020 069 623

## Description

### TECHNICAL FIELD

The present invention relates to a liquid formulation comprising cilastatin or a pharmaceutically acceptable salt thereof. This invention also relates to a pharmaceutical product comprising a package and a liquid formulation placed in the package, the liquid formulation comprising cilastatin or a pharmaceutically acceptable salt thereof.

### BACKGROUND ART

Cilastatin is known to have inhibitory activity against DHP-I (dehydropeptidase-I) which is a metabolic enzyme present in renal proximal tubular brush-border membranes. Cilastatin is clinically used with the carbapenem-based antibiotic imipenem for the purpose of preventing inactivation of imipenem by DHP-I.

Cilastatin is also known to have other various activities. For example, PTL 1 discloses an inhibitor for megalin-mediated renal injuries, comprising cilastatin. PTL 2 discloses utilization of cilastatin in inhibiting renal injuries.

There are reports on the stabilization of a composition comprising cilastatin. For example, PTL 3 discloses a pharmaceutical composition comprising cilastatin and imipenem in combination with a chelating agent.
PTL 4 relates to a parenteral preparation comprising EDTA and water for injection for use as diluent, and further one or more antibiotic agents, e.g., cilastatin. PTL 5 relates to a cilastatin treatment as such, i.e., a method for preventing or treating sepsis in a mammalian subject. PTL 6 discloses a sterile powder preparation of imipenem-cilastatin sodium for injection, comprising an antibacterial agent, an inhibitor, a solubilizer and a pH adjusting agent, wherein the antibacterial agent is imipenem, the inhibitor is cilastatin sodium.

### CITATION LIST

### PATENT LITERATURES

PTL 1: International Patent Publication No. WO 2015/111666
PTL 2: International Patent Publication No. WO 2019/208777
PTL 3: International Patent Publication No. WO 2010/092446
PTL 4: WO 2018/025248 A1
PTL 5: US 2020/069623 A1
PTL 6: CN 106 176 722 A

### SUMMARY OF INVENTION

### TECHNICAL PROBLEM

It is considered that when cilastatin or a pharmaceutically acceptable salt thereof is administered by infusion or injection, cilastatin or the like is provided in the form of a dry powder formulation or a liquid formulation. It can be predicted that the dry powder formulation will be superior in stability. In contrast, the liquid formulation is superior in that it can be instantly administered without taking a dissolution step, but is questionable from a stability perspective. In fact, the present inventors confirmed that as time passes, a liquid formulation comprising cilastatin or a pharmaceutically acceptable salt thereof increases in the degree of coloration, and/or decreases in the content of cilastatin or a salt thereof.

Therefore, an object of the present invention is to enhance the stability of a liquid formulation comprising cilastatin or a pharmaceutically acceptable salt thereof.

### SOLUTION TO PROBLEM

The present inventors have conducted intensive studies to achieve the aforementioned object, and as a result, found that particular compounds are useful as stabilizing agents for a liquid formulation comprising cilastatin or a pharmaceutically acceptable salt thereof.

The present invention as defined in the appended claims includes the following embodiments.
[1] A pharmaceutical product comprising:
   a) a package, and a liquid formulation placed in the package,
      wherein the liquid formulation comprises 0.25 to 25 w/v% of cilastatin or a pharmaceutically acceptable salt thereof, and 0.01 to 1 w/v% of at least one selected from the group consisting of pyrosulfurous acid, sulfurous acid, substituted or unsubstituted phenol, cysteine, and pharmaceutically acceptable salts thereof,
      wherein a headspace in the package is purged with inert gas; or
   b) a package, and a liquid formulation placed in the package,
      wherein the liquid formulation comprises 0.25 to 25 w/v% of cilastatin or a pharmaceutically acceptable salt thereof, 0.1 to 2 w/v% of nicotinamide or a pharmaceutically acceptable salt thereof, and 0.01 to 1 w/v% of a chelating agent,
      wherein a headspace in the package is purged with inert gas.
[2] The pharmaceutical product as set forth in [1], wherein the liquid formulation does not contain NaHCO₃.
[3] The pharmaceutical product as set forth in [1] or [2], wherein the liquid formulation of a) further comprises 0.01 to 1 w/v% of a chelating agent.
[4] The pharmaceutical product as set forth in any one of [1] to [3], wherein the chelating agent is selected from the group consisting of ethylenediamine tetraacetic acid, diethylenetriamine pentaacetic acid, hydroxyethylene diamine triacetic acid, nitrilotriacetic acid, O,O'-bis(2-aminoethyl)ethylene glycol-N,N,N',N'-tetraacetic acid, trans-1,2-diaminocyclohexane-N,N,N',N'-tetraacetic acid, citric acid, and pharmaceutically acceptable salts thereof.
[5] A pharmaceutical product comprising:
   a package, and a liquid formulation placed in the package,
   wherein a headspace in the package is purged with inert gas, and
   wherein the liquid formulation comprises 0.25 to 25 w/v% of cilastatin or a pharmaceutically acceptable salt thereof, and 0.01 to 1 w/v% of citric acid or a pharmaceutically acceptable salt thereof, but does not contain imipenem.
[6] The pharmaceutical product as set forth in [5 ], wherein the liquid formulation does not contain NaHCO₃.

### ADVANTAGEOUS EFFECTS OF INVENTION

The present invention can enhance the stability of a liquid formulation comprising cilastatin or a pharmaceutically acceptable salt thereof. To be specific, this invention can suppress an increase in the degree of coloration of a liquid formulation comprising cilastatin or a pharmaceutically acceptable salt thereof. In a certain embodiment, this invention can suppress a decrease in the content of cilastatin or a pharmaceutically acceptable salt thereof in the liquid formation.

As referred to herein, the coloration of the liquid formation means that the liquid formulation develops a color such as a yellow or brown color as time passes. The degree of coloration can be evaluated through measuring an absorbance at a particular wavelength.

### BRIEF DESCRIPTION OF DRAWINGS

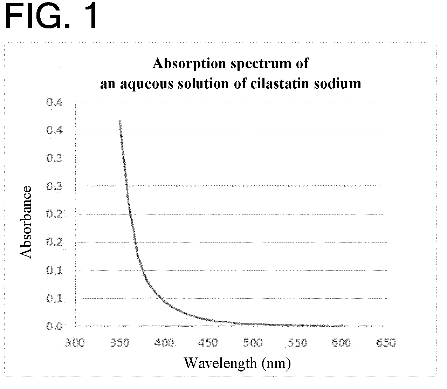

[FIG. 1] FIG. 1 depicts the absorption spectrum of an aqueous solution of cilastatin sodium.

### DESCRIPTION OF EMBODIMENTS

Hereunder, the liquid formulation and pharmaceutical product of the present invention will be described.

### (Liquid formulation)

In one embodiment, the present invention is directed to a liquid formulation comprising cilastatin or a pharmaceutically acceptable salt thereof. The liquid formation not only comprises cilastatin or a pharmaceutically acceptable salt thereof and a stabilizing agent, but also comprises, as a liquid medium, a pharmaceutically acceptable liquid such as water, an alcohol (e.g., ethanol, polyethylene glycol, propylene glycol), or a mixture thereof. The liquid formation may be a solution or a suspension, or may comprise a microcapsule. The liquid formation can be in the form of a liquid pharmaceutical, an injectable, or a spray.

A preferred form is an injectable.

### (Cilastatin)

The liquid formulation of the present invention comprises cilastatin or a pharmaceutically acceptable salt thereof.

Cilastatin refers to (Z)-7-[[(R)-2-amino-2-carboxyethyl]thio]-2-[[[(S)-2,2-dimethylcyclopropyl]carbonyl]amino]-2-heptenoic acid. For the sake of confirmation, with regard to a compound such as cilastatin as referred to herein, when such a compound produces a hydrate, use of the hydrate of the compound is also included within the scope of this invention.

Examples of a pharmaceutically acceptable salt of cilastatin include, but are not limited to, alkali metal salts, such as lithium salt, sodium salt and potassium salt; alkali earth metal salts, such as magnesium salt and calcium salt; transition metal salts, such as zinc salt, iron salt, cobalt salt and cupper salt; aluminum salt; organic amine salts, such as choline salt, ethanolamine salt, trimethylamine salt, triethylamine salt, dicyclohexylamine salt, dibenzylamine salt, phenethylbenzylamine salt, procaine salt, morpholine salt, pyridine salt, piperidine salt, piperadine salt and N-ethylpiperidine salt; ammonium salt; and basic amino acid salts, such as lysine salt and arginine salt. A preferred salt is a sodium salt. For the sake of confirmation, the scope of the term "pharmaceutically acceptable salts" as referred to herein also includes hydrates of the salts. When the liquid formulation of the present invention comprises a salt of cilastatin, the liquid formulation may comprise only a single type of the aforementioned salts or may comprise two or more types thereof.

As cilastatin or a pharmaceutically acceptable salt thereof, use can be made of, for example, a commercially available product, or a product produced and obtained by *a per se* known method or by a method pursuant to a known method. For example, commercially available cilastatin sodium products can be acquired from ACS Dobfer (Milan, Italy), Dongkook Lifesceience (Seoul, Korea), or the like.

The content of cilastatin or a pharmaceutically acceptable salt thereof in the liquid formulation of the present invention can be such an amount that cilastatin or the salt thereof can be provided in an effective amount to produce the desired pharmacological effects. For the purpose of inhibition of renal injuries, an exemplary daily dose of cilastatin or a salt thereof in an adult is from 1.0 to 2.0 g, or from 1.0 to 1.5 g. The liquid formulation can be administered in a single dose or in divided doses to achieve such a daily dose. The liquid formulation may also be administered using an intermittent dosing regimen, such as alternate-day or every three day regimen. The content of cilastatin or a pharmaceutically acceptable salt thereof in the liquid formulation of this invention is in the range of from 0.25 to 25 w/v%.

Examples of stabilizing agents used in combination with cilastatin or a pharmaceutically acceptable salt thereof will be described below.

### (Cysteine)

In one embodiment, the liquid formulation of the present invention comprises not only cilastatin or a pharmaceutically acceptable salt thereof, but also cysteine or a pharmaceutically acceptable salt thereof. Cysteine exists in two stereoisomers: L- and D-forms. The liquid formulation of this invention comprises L-cysteine, D-cysteine, or both of them. The liquid formulation of this invention preferably comprises L-cysteine.

Examples of a pharmaceutically acceptable salt of cysteine include, but are not limited to, alkali metal salts, such as lithium salt, sodium salt and potassium salt; alkali earth metal salts, such as magnesium salt and calcium salt; transition metal salts, such as zinc salt, iron salt, cobalt salt and cupper salt; aluminum salt; organic amine salts, such as choline salt, ethanolamine salt, trimethylamine salt, triethylamine salt, dicyclohexylamine salt, dibenzylamine salt, phenethylbenzylamine salt, procaine salt, morpholine salt, pyridine salt, piperidine salt, piperadine salt and N-ethylpiperidine salt; ammonium salt; basic amino acid salts, such as lysine salt and arginine salt; inorganic acid salts, such as hydrochloride, sulfate, and phosphate; organic acid salts, such as formate, acetate, maleate, fumarate, and tartrate. Preferred salts of cysteine are hydrochloride, sulfate, phosphate, formate, and acetate, with hydrochloride being more preferred. When the liquid formulation of the present invention comprises a salt of cysteine, the liquid formulation may comprise only a single type of the aforementioned salts or may comprise two or more types thereof.

Cysteine or a pharmaceutically acceptable salt thereof is capable of enhancing the stability of the liquid formulation of the present invention. The content of cysteine or a pharmaceutically acceptable salt thereof in the liquid formulation of this invention is in the range of from 0.01 to 1 w/v%.

Preferably, the liquid formation is placed in a package, and a headspace in the package is purged with inert gas. The liquid formation preferably comprises a chelating agent.

In one preferred embodiment, the liquid formation comprises a chelating agent and is placed in a package, and a headspace in the package is purged with inert gas.

### (Pyrosulfurous acid, sulfurous acid, phenol)

In one embodiment, the liquid formulation of the present invention comprises not only cilastatin or a pharmaceutically acceptable salt thereof, but also at least one selected from the group consisting of pyrosulfurous acid, sulfurous acid, substituted or unsubstituted phenol, and pharmaceutically acceptable salts thereof.

Examples of pharmaceutically acceptable salts of sulfurous acid, pyrosulfurous acid, or substituted or unsubstituted phenol are the same as described above with regard to salts of cilastatin. Preferred examples of such salts are sodium sulfite (Na₂SO₃), sodium bisulfite (NaHSO₃), and sodium pyrosulfite (Na₂S₂O₅). When the liquid formulation of the present invention comprises a salt of sulfurous acid, pyrosulfurous acid, or substituted or unsubstituted phenol, the liquid formulation may comprise only a single type of the aforementioned salts or may comprise two or more types thereof.

Examples of substituted or unsubstituted phenol include, but are not limited to, phenol, catechol, resorcinol, hydroquinone, cresol, xylol, hydroxyquinol, phloroglucinol, and pyrogallol, with phenol being preferred.

The aforementioned compounds used as stabilizing agents are capable of enhancing the stability of the liquid formulation of the present invention. The content of such an aforementioned compound in the liquid formulation of this invention is in the range of from 0.01 to 1 w/v%.

In the present invention, the liquid formation is placed in a package, and a headspace in the package is purged with inert gas. Preferably, the liquid formation comprises a chelating agent.

In a preferred embodiment, the liquid formation comprises a chelating agent and is placed in a package, and a headspace in the package is purged with inert gas.

### (Nicotinamide)

In one embodiment, the liquid formulation of the present invention comprises not only cilastatin or a pharmaceutically acceptable salt thereof, but also nicotinamide or a pharmaceutically acceptable salt thereof.

Examples of a pharmaceutically acceptable salt of nicotinamide include inorganic acid salts, such as hydrochloride, sulfate, and phosphate; organic acid salts, such as formate, acetate, maleate, fumarate, and tartrate. Preferred salts are hydrochloride and acetate. When the liquid formulation of the present invention comprises a salt of nicotinamide, the liquid formulation may comprise only a single type of the aforementioned salts or may comprise two or more types thereof.

Nicotinamide or a pharmaceutically acceptable salt thereof is capable of enhancing the stability of the liquid formulation of the present invention. In one embodiment, the content of nicotinamide or a pharmaceutically acceptable salt thereof in the liquid formulation of this invention is in the range of from 0.1 to 2 w/v%.

The liquid formation preferably comprises a chelating agent. Also, the liquid formation is placed in a package, and a headspace in the package is purged with inert gas. In a preferred embodiment, the liquid formation comprises a chelating agent and is placed in a package, and a headspace in the package is purged with inert gas.

### (Chelating agent)

In one embodiment, the liquid formulation of the present invention may comprise a chelating agent in the absence or presence of the cilastatin-stabilizing agent(s) as mentioned above. Preferably, the chelating agent is selected from the group consisting of ethylenediamine tetraacetic acid, diethylenetriamine pentaacetic acid, hydroxyethylene diamine triacetic acid, nitrilotriacetic acid, O,O'-bis(2-aminoethyl)ethylene glycol-N,N,N',N'-tetraacetic acid, trans-1,2-diaminocyclohexane-N,N,N',N'-tetraacetic acid, citric acid, and pharmaceutically acceptable salts thereof. More preferred chelating agents are ethylenediamine tetraacetic acid, citric acid, or pharmaceutically acceptable salts thereof.

The chelating agent is capable of enhancing the stability of the liquid formulation of the present invention. The content of the chelating agent in the liquid formulation of this embodiment is in the range of from 0.01 to 1 w/v%.

The liquid formation is placed in a package, and a headspace in the package is purged with inert gas. (pH)

The pH of the liquid composition of the present invention is in the range of preferably from 5 to 8, more preferably from 5.5 to 7.5. When the pH is less than 5, the effects of this invention may not be fully obtained.

### (Pharmaceutical product)

The pharmaceutical product of the present invention comprises a package and a liquid formulation placed in the package. The liquid formation can be any of the aforementioned liquid formulations comprising cilastatin or a pharmaceutically acceptable salt thereof. A headspace in the package is purged with inert gas.

The inert gas can be any known gas such as nitrogen gas or argon gas, but is preferably nitrogen gas. The headspace in a package preferably contains no oxygen at all but may contain a small amount of oxygen. The oxygen concentration in the headspace is preferably not more than 1.0% by volume, more preferably not more than 0.2% by volume.

### (Package)

The form and material of a package in which the liquid formulation of the present invention is placed are not particularly limited. The material of the package can be, for example, glass, a resin, or the like, and the form of the package can be, for example, a bag, a glass bottle, an ampule, or a syringe barrel. Preferably, the package is a glass vial.

### (Other components)

The liquid formulation of the present invention may further comprise other components commonly used in pharmaceuticals, such as pH adjustor, solubilizer, suspending agent, emulsifier, isotonizer, soothing agent, antiseptic, and antioxidant.

In a preferred embodiment, the liquid formulation and pharmaceutical product of the present invention does not contain imipenem. In another preferred embodiment, the liquid formulation and pharmaceutical product of this invention does not contain NaHCO₃. The scope of meaning of the term "not contain(ing)" as used herein in relation to a particular component covers not only an absolutely zero content of the particular component, but also contents thereof below the limit of detection, such as less than 0.001 w/v%, less than 0.01 w/v%, less than 0.1 w/v%, or less than 0.2 w/v%.

### (Numerical range)

For the purpose of clarification, all numerical ranges defined herein by lower and upper limits include their lower and upper limits. For example, the range defined as "from 1.0 to 2.0 g" includes its lower and upper limits.

### EXAMPLES

Hereunder, the present invention will be described by way of working examples.

### (Test 1) Deterioration test of a cilastatin-containing liquid formulation

A deterioration test of a liquid formulation was performed using cilastatin sodium.

The cilastatin sodium used was acquired from ACSD. In a preliminary investigation, a solution obtained by dissolving cilastatin sodium in deionized water had a slightly yellow color at the time of preparation. This solution changed its color to yellow after storage at 45°C for 23 days, and then to brown after storage for 65 days. The content of cilastatin decreased slightly.

In consideration of the results of the preliminary investigation, more detailed investigation was performed using cilastatin sodium. To be specific, an aqueous solution was prepared by dissolving cilastatin sodium in deionized water at a concentration of 200 mg/mL, and bottled up in a glass vial to be sealed, and then the glass vial was heated at 80°C for 4 hours (to obtain a heated sample). Separately, an aqueous solution of cilastatin sodium prepared in the same way was filled in another glass vial, and the headspace in the glass vial was purged with nitrogen. Then, the glass vial was sealed and heated at 80°C for 4 hours (to obtain a nitrogen-purged, heated sample). The aqueous solution before heating and the two samples prepared above were measured for absorbance, which served as an index for the degree of coloration, and also analyzed by HPLC to confirm the content of cilastatin. The aqueous solutions used in all the cases had a pH of 6.79 at the time of preparation.

Considering that an aqueous solution of cilastatin sodium (200 mg/mL) has such an absorbance curve as shown in FIG. 1, a wavelength of 400 nm was used to measure absorbance. According to visual observation, the aqueous solutions had a slightly yellow appearance.

The analysis conditions are detailed below.
Absorbance measurement conditions:

| | |
|---|---|
| Equipment: | Hitachi U-3900 |
| Optical path length: | 1 cm |
| Cilastatin sodium: | Aqueous solution of 200 mg/mL cilastatin sodium |

HPLC analysis conditions:

| | |
|---|---|
| Detector: | Ultraviolet absorptiometer (measurement wavelength: 216 nm) |
| Column: | 1.7 µm octadecylsilylated silica gel column (Acquity BEH C18, produced by Waters), 2.1 mm I.D., 50 mm long |
| Column temperature: | 40°C |
| Mobile phase: | 25 mM phosphate buffer containing 9.5% acetonitrile (pH 4.25) |
| Amount of sample injected: | 5 µL (prepared to 100 µg/mL) |
| Flow rate: | 0.3 mL/min. |
| Retention time: | Cilastatin (about 2.6 min.) |

The results are shown in the table given below. While there were great variations in absorbance, a decrease in the content of cilastatin was relatively small. Nitrogen purging produced a certain stabilization effect.

**[Table 1]**

| | Absorbance | Cilastatin content |
|---|---|---|
| Before heating | 0.040 | 98.99% |
| Heated sample | 0.203 | 98.85% |
| N₂-purged, heated sample | 0.166 | 99.15% |

### (Test 2) Effects of antioxidants

The effects of different antioxidants were investigated using samples prepared by adding any of the known antioxidants, sodium sulfite, sodium pyrosulfite, or ascorbic acid, to a cilastatin-containing liquid formulation. The effect of nitrogen purging was also investigated.

To be specific, cilastatin sodium was dissolved in deionized water at a concentration of 200 mg/mL to prepare an aqueous solution. A portion (0.5 mL) of the aqueous solution was bottled up in a glass vial to be sealed, and the glass vial was stored at 45°C for 18 days (control). Separately, an antioxidant (1 mg/mL sodium sulfite, 1 mg/mL sodium pyrosulfite, or 1 mg/mL ascorbic acid) was added to another portion (0.5 mL) of the prepared aqueous solution, and the mixed solution was bottled up in a glass vial to be sealed, and sotred at 45°C for 18 days. Thus, a total of four different samples were obtained in the aforementioned ways.

Also, nitrogen-purged samples corresponding to the aforementioned four samples were prepared. To be specific, four different nitrogen-purged samples were prepared by following the same procedures as mentioned above except that after the aqueous solution of cilastatin sodium was placed in a glass vial, the headspace in the glass vial was purged with nitrogen.

These eight samples measured for absorbance, which served as an index for the degree of coloration, and also analyzed by HPLC to confirm the content of cilastatin. The analysis conditions were the same as in Test 1, but absorbance measurements were performed after the aqueous solutions were diluted if required (the degree of dilution was the same for all the samples). The aqueous solutions used to prepare all the samples had a pH of from 6.50 to 6.73 at the time of preparation.

The results are shown in the table given below.

**[Table 2]**

| | Absorbance | | Cilastatin content w/N₂-purge |
|---|---|---|---|
| | w/o N₂-purge | w/N₂-purge | |
| Control | 0.966 | 0.437 | 98.36% |
| Na₂SO₃ | 1.256 | 0.192 | 98.23% |
| Na₂S₂O₅ | 1.252 | 0.304 | 98.42% |
| Ascorbic acid | 4.302 | 3.425 | 98.02% |

Stabilization of aqueous solutions was achieved only in the case of using some of the antioxidants. Moreover, it was found that combination of the antioxidant with nitrogen purging is important for stabilization.

### (Test 3) Effects of additives

Experimentation was performed by following the same procedures as in Test 2 except that antioxidants were replaced with other additives. However, the storage period was set to four weeks. Also, the additives added to the aqueous solutions were sodium sulfite (1 mg/mL), sodium pyrosulfite (1 mg/mL), L-cysteine (1 mg/mL), disodium ethylenediamine tetraacetate (EDTA) (1 mg/mL), NaHCO₃ (8 mg/mL), phenol (1 mg/mL), or nicotinamide (10 mg/mL). The aqueous solutions used to obtain the samples had a pH of from 6.45 to 7.04 at the time of preparation.

The results are shown in the table given below.

**[Table 3]**

| | Absorbance | | Cilastatin content w/ N₂-purge |
|---|---|---|---|
| | w/o N₂-purge | w/ N₂-purge | |
| Control | 1.122 | 0.471 | 98.36% |
| Na₂SO₃ | 1.538 | 0.226 | 98.28% |
| Na₂S₂O₅ | 1.424 | 0.192 | 98.42% |
| L-cysteine | 0.674 | 0.327 | 98.45% |
| Phenol | 1.246 | 0.324 | 98.91% |
| NaHCO₃ | 1.467 | 0.590 | 97.34% |
| Nicotinamide | 1.216 | 0.495 | 98.99% |
| EDTA 2Na | 0.446 | 0.368 | 98.81% |

Stabilization of aqueous solutions was observed only in the cases of using L-cysteine or disodium EDTA. Further, in nitrogen-purged samples, the effect of using L-cysteine was higher than that of using disodium EDTA. No clear stabilization was achieved in the case of using nicotinamide. While conventional formulations comprising cilastatin and imipenem contain NaHCO₃, addition of NaHCO₃ in the present invention rather resulted in decreased stabilization of the aqueous solution of cilastatin sodium. The other additives showed superior stabilization effects when combined with nitrogen purging.

### (Test 4) Effects of chelating agents

Experimentation was performed by following the same procedures as in Test 2 except that antioxidants were replaced with chelating agents. However, the storage period was set to four weeks. The chelating agents added to the aqueous solutions were disodium ethylenediamine tetraacetate (EDTA) (1 mg/mL) or citric acid (1 mg/mL). The aqueous solutions used to prepare all the samples had a pH of from 5.89 to 7.04 at the time of preparation.

The results are shown in the table given below.

**[Table 4]**

| | Absorbance | | Cilastatin content w/ N₂-purge |
|---|---|---|---|
| | w/o N₂-purge | w/ N₂-purge | |
| Control | 1.122 | 0.471 | 98.36% |
| Citric acid | 0.578 | 0.316 | 98.41% |
| EDTA 2Na | 0.446 | 0.368 | 98.81% |

Stabilization of the aqueous solutions of cilastatin sodium was observed when the chelating agents were used.

### (Test 5) Effects of combined use of a chelating agent with additives

Experimentation was performed by following the same procedures as in Test 2 except that antioxidants were replaced with a combination of a chelating agent with other additives. However, the storage period was set to four weeks. The chelating agent added to aqueous solutions was disodium ethylenediamine tetraacetate (EDTA) (1 mg/mL). A sample prepared with addition of only the chelating agent was used as a control. The additives combined with the chelating agent were sodium pyrosulfite (1 mg/mL), L-cysteine (1 mg/mL), phenol (1 mg/mL), or nicotinamide (10 mg/mL). The aqueous solutions used to prepare all the samples had a pH of from 6.38 to 7.04 at the time of preparation.

The results are shown in the table given below.

**[Table 5]**

| | Absorbance | | Cilastatin content w/ N₂-purge |
|---|---|---|---|
| | w/o N₂-purge | w/ N₂-purge | |
| EDTA 2Na only (control) | 0.446 | 0.368 | 98.81% |
| EDTA 2Na + Na₂S₂O₅ | 0.406 | 0.233 | 98.80% |
| EDTA 2Na + L-cysteine | 0.472 | 0.209 | 98.84% |
| EDTA 2Na + phenol | 0.470 | 0.274 | 99.01 % |
| EDTA 2Na + nicotinamide | 0.486 | 0.261 | 98.60% |

Apparently superior stabilization of the aqueous solution was observed in the case of using a combination of sodium pyrosulfite and the chelating agent. Also, combinations of L-cysteine, phenol or nicotinamide with the chelating agent showed a high stabilization effect when further combined with nitrogen purging.

### INDUSTRIAL APPLICABILITY

The present invention can provide a liquid formulation with enhanced stability comprising cilastatin or a pharmaceutically acceptable salt thereof.

## Claims

1. A pharmaceutical product comprising:
a) a package, and a liquid formulation placed in the package,
wherein the liquid formulation comprises 0.25 to 25 w/v% of cilastatin or a pharmaceutically acceptable salt thereof, and 0.01 to 1 w/v% of at least one selected from the group consisting of pyrosulfurous acid, sulfurous acid, substituted or unsubstituted phenol, cysteine, and pharmaceutically acceptable salts thereof,
wherein a headspace in the package is purged with inert gas; or
b) a package, and a liquid formulation placed in the package,
wherein the liquid formulation comprises 0.25 to 25 w/v% of cilastatin or a pharmaceutically acceptable salt thereof, 0.1 to 2 w/v% of nicotinamide or a pharmaceutically acceptable salt thereof, and 0.01 to 1 w/v% of a chelating agent,
wherein a headspace in the package is purged with inert gas.

2. The pharmaceutical product according to claim 1, wherein the liquid formulation does not contain NaHCO₃.

3. The pharmaceutical product according to claim 1 or 2, wherein the liquid formulation of a) further comprises 0.01 to 1 w/v% of a chelating agent.

4. The pharmaceutical product according to any one of claims 1 to 3, wherein the chelating agent is selected from the group consisting of ethylenediamine tetraacetic acid, diethylenetriamine pentaacetic acid, hydroxyethylene diamine triacetic acid, nitrilotriacetic acid, O,O'-bis(2-aminoethyl)ethylene glycol-N,N,N',N'-tetraacetic acid, trans-1,2-diaminocyclohexane-N,N,N',N'-tetraacetic acid, citric acid, and pharmaceutically acceptable salts thereof.

5. A pharmaceutical product comprising:
a package, and a liquid formulation placed in the package,
wherein a headspace in the package is purged with inert gas, and
wherein the liquid formulation comprises 0.25 to 25 w/v% of cilastatin or a pharmaceutically acceptable salt thereof, and 0.01 to 1 w/v% of citric acid or a pharmaceutically acceptable salt thereof , but does not contain imipenem.

6. The pharmaceutical product according to claim 5, wherein the liquid formulation does not contain NaHCO₃.

## Patentansprüche

1. Ein pharmazeutisches Produkt, umfassend:
a) eine Verpackung und eine in der Verpackung befindliche flüssige Formulierung,
wobei die flüssige Formulierung 0,25 bis 25 Gew./Vol.-% Cilastatin oder ein pharmazeutisch verträgliches Salz davon und 0,01 bis 1 Gew./Vol.-% mindestens eines, ausgewählt aus der Gruppe bestehend aus Pyroschwefelsäure, Schwefelsäure, substituiertem oder unsubstituiertem Phenol, Cystein und pharmazeutisch verträglichen Salzen davon, umfasst,
wobei ein Kopfraum in der Verpackung mit Inertgas gespült ist; oder
b) eine Verpackung und eine in der Verpackung befindliche flüssige Formulierung,
wobei die flüssige Formulierung 0,25 bis 25 Gew./Vol.-% Cilastatin oder ein pharmazeutisch verträgliches Salz davon, 0,1 bis 2 Gew./Vol.-% Nicotinamid oder ein pharmazeutisch verträgliches Salz davon und 0,01 bis 1 Gew./Vol.-% eines Chelatbildners umfasst,
wobei ein Kopfraum in der Verpackung mit Inertgas gespült ist.

2. Das pharmazeutische Produkt nach Anspruch 1, wobei die flüssige Formulierung kein NaHCO₃ enthält.

3. Das pharmazeutische Produkt nach Anspruch 1 oder 2, wobei die flüssige Formulierung aus a) ferner 0,01 bis 1 Gew./Vol.-% eines Chelatbildners umfasst.

4. Das pharmazeutische Produkt nach einem der Ansprüche 1 bis 3, wobei der Chelatbildner ausgewählt ist aus der Gruppe bestehend aus Ethylendiamintetraessigsäure, Diethylentriaminpentaessigsäure, Hydroxyethylendiamintriessigsäure, Nitrilotriessigsäure, O,O'-Bis(2-aminoethyl)ethylenglykol-N,N,N',N'-tetraessigsäure, trans-1,2-Diaminocyclohexan-N,N,N',N'-tetraessigsäure, Zitronensäure und pharmazeutisch verträglichen Salzen davon.

5. Ein pharmazeutisches Produkt, umfassend:
eine Verpackung und eine in der Verpackung befindliche flüssige Formulierung,
wobei ein Kopfraum in der Verpackung mit Inertgas gespült ist und
wobei die flüssige Formulierung 0,25 bis 25 Gew./Vol.-% Cilastatin oder ein pharmazeutisch verträgliches Salz davon und 0,01 bis 1 Gew./Vol.-% Zitronensäure oder ein pharmazeutisch verträgliches Salz davon umfasst, jedoch kein Imipenem enthält.

6. Das pharmazeutische Produkt nach Anspruch 5, wobei die flüssige Formulierung kein NaHCO₃ enthält.

## Revendications

1. Produit pharmaceutique comprenant :
a) un emballage et une formulation liquide placée dans l'emballage,
dans lequel la formulation liquide comprend 0,25 à 25 % p/v de cilastatine ou d'un sel pharmaceutiquement acceptable de celle-ci, et 0,01 à 1 % p/v d'au moins un élément choisi dans le groupe constitué par l'acide pyrosulfureux, l'acide sulfureux, le phénol substitué ou non substitué, la cystéine et les sels pharmaceutiquement acceptables de ceux-ci,
dans lequel un espace libre dans l'emballage est purgé par un gaz inerte ; ou
b) un emballage, et une formulation liquide placée dans l'emballage,
dans lequel la formulation liquide comprend 0,25 à 25 % p/v de cilastatine ou d'un sel pharmaceutiquement acceptable de celle-ci, 0,1 à 2 % p/v de nicotinamide ou d'un sel pharmaceutiquement acceptable de celui-ci, et 0,01 à 1 % p/v d'un agent chélatant,
dans lequel un espace libre dans l'emballage est purgé par un gaz inerte.

2. Produit pharmaceutique selon la revendication 1, dans lequel la formulation liquide ne contient pas de NaHCO₃.

3. Produit pharmaceutique selon la revendication 1 ou 2, dans lequel la formulation liquide de a) comprend en outre 0,01 à 1 % p/v d'un agent chélatant.

4. Produit pharmaceutique selon l'une quelconque des revendications 1 à 3, dans lequel l'agent chélatant est choisi dans le groupe constitué par l'acide éthylènediamine tétraacétique, l'acide diéthylènetriamine pentaacétique, l'acide hydroxyéthylène diamine triacétique, l'acide nitrilotriacétique, l'acide O,O'-bis(2-aminoéthyl)éthylène glycol-N,N,N',N'-tétraacétique, l'acide trans-1,2-diaminocyclohexane-N,N,N',N'-tétraacétique, l'acide citrique et les sels pharmaceutiquement acceptables de ceux-ci.

5. Produit pharmaceutique comprenant :
un emballage, et une formulation liquide placée dans l'emballage,
dans lequel un espace libre dans l'emballage est purgé par un gaz inerte, et
dans lequel la formulation liquide comprend 0,25 à 25 % p/v de cilastatine ou d'un sel pharmaceutiquement acceptable de celle-ci, et 0,01 à 1 % p/v d'acide citrique ou d'un sel pharmaceutiquement acceptable de celui-ci, mais ne contient pas d'imipénem.

6. Produit pharmaceutique selon la revendication 5, dans lequel la formulation liquide ne contient pas de NaHCO₃.
